# EUROPEAN PATENT APPLICATION

(11) **EP 3 249 385 A1**
(43) Date of publication of application: **29.11.2017**
(21) Application number: 17168667.8
(22) Date of filing: 28.04.2017
(51) Int. Cl.: G01N 21/3563, B07C 5/342, G01N 33/44, B29B 17/02, G01J 3/42, C08J 11/04, G01N 21/359, G01N 21/85, G01N 21/94

(54) **SPECIFIC BROMINE-BASED FLAME RETARDANT DETERMINATION METHOD AND DETERMINATION APPARATUS**

(30) Priority: 27.05.2016 JP 2016106540
(71) Applicant: Panasonic Intellectual Property Management Co., Ltd., Osaka-shi, Osaka 540-6207 (JP)
(72) Inventor: HAMADA, Shingo, Osaka-shi, Osaka 540-6207 (JP); MIYAMAE, Nobuhiko, Osaka-shi, Osaka 540-6207 (JP); YAMAGUCHI, Naoshi, Osaka-shi, Osaka 540-6207 (JP)
(74) Representative: Vigand, Philippe

(57) **Abstract**

A resin (2, 2a, 2b) is irradiated with an infrared ray (3), and a reflected ray (4) from the resin (2, 2a, 2b) irradiated with the infrared ray (3) is received by an infrared detection unit (8). In a reflection or absorption spectrum obtained by the reflected ray (4), a difference of a reflection intensity in a spectrum between a first wave number band of 1340 cm⁻¹ to 1350 cm⁻¹, inclusive, and a second wave number band of 1300 cm⁻¹ to 1340 cm⁻¹, inclusive, is calculated. It is determined whether or not a specific bromine-based flame retardant is contained in the resin (2, 2a, 2b), by using the calculated difference of reflection intensity in the spectrum.

## Description

### BACKGROUND

### 1. Technical Field

The disclosure relates to a specific bromine-based flame retardant determination method and a determination apparatus that determine whether or not a resin contains a specific bromine-based flame retardant.

### 2. Description of the Related Art

Environmental problems in global scale, such as global warming or exhaustion of resources occur by economic activities of mass consumption and mass disposal type.

In such a situation, in Japan, the home appliance recycling law has been enforced from April 2001, in order to construct a society of a resource recycling. With the home appliance recycling law, people are obligated to recycle the used home appliances (air conditioner, television, refrigerator, freezer, washing machine, clothes dryer, and the like).

Thus, the used home appliance is crushed, and then is selected and recovered for each material by using magnetism, wind power, vibration, and the like in a home appliance recycling factory, and thus is recovered as a recycled material.

In Directive on "Restriction of the use of certain Hazardous Substances (abbreviation: RoHS Directive)", which took effect on July 2006 by the European Union (EU), using polybromobiphenyl (PBB) or polybromodiphenyl ether (PBDE) (set as specific bromine below) in home appliances is restricted.

Among resins used in home appliances, there is a resin using a flame retardant (hereinafter, specific bromine-based flame retardant) containing specific bromine in order to take flame retardance.

A product using a resin containing the above substance as a recycled material is a regulation object by RoHS Directive.

A bromine-based flame retardant (hereinafter, non-specific bromine-based flame retardant) which contains bromine other than the specific bromine is also used in home appliances, but this is not an object of RoHS regulation.

Thus, determination of whether or not a resin of a recycled material contains a specific bromine-based flame retardant is required.

As a determination method of a bromine-based flame retardant and a recycling device using the same in the related art, there is a technology using a near-infrared spectroscopy which uses a near-infrared ray (light having a wavelength of 2.5 µm or less) (see Patent Literature 1 (Japanese Patent No. 5290466)). This method is a method in which an absorption spectrum of C-H bond is detected, and it is evaluated whether or not a peak position thereof is shifted, and thus it is determined whether or not a bromine-based flame retardant is provided.

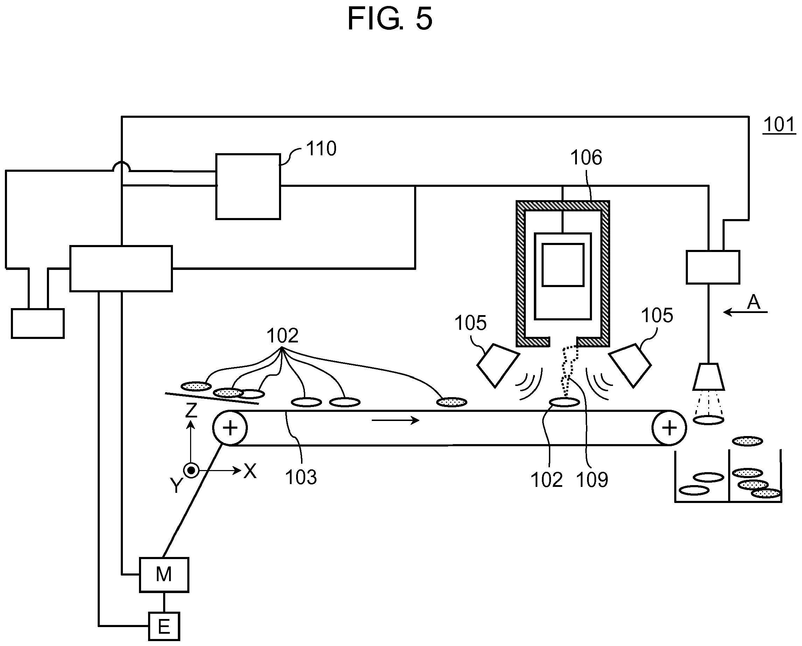

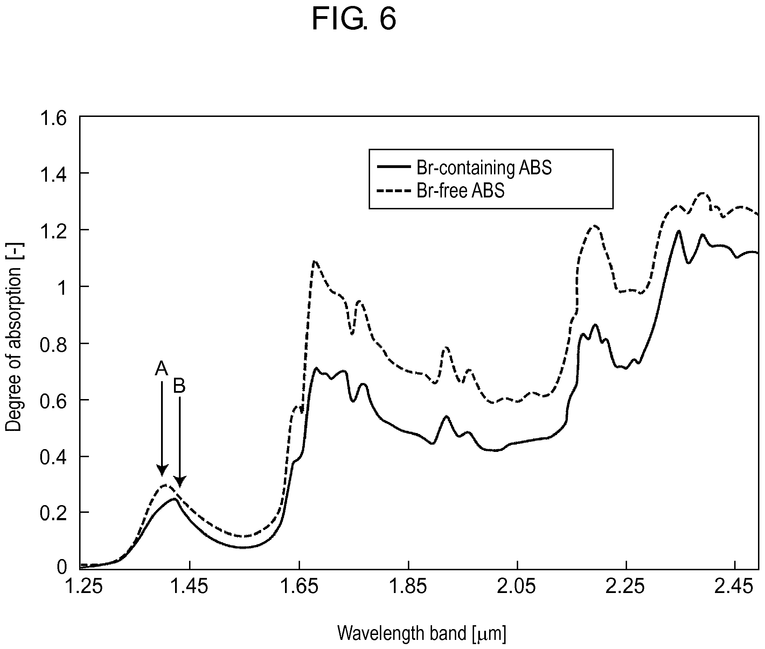

FIG. 5 is a schematic diagram illustrating a determination apparatus of the related art disclosed in Patent Literature 1. FIG. 6 illustrates a graph of an absorption spectrum used for determining whether or not a bromine-based flame retardant is contained in an ABS (acrylonitrile ·butadiene ·styrene) resin disclosed in Patent Literature 1.

In FIG. 5, a reference sign of 101 indicates a bromine-based flame retardant determination apparatus. Bromine-based flame retardant determination apparatus 101 includes halogen lamp 105, near-infrared ray detection device 106, conveyor 103, and arithmetic processing device (arithmetic processor) 110. Halogen lamp 105 is an example of an irradiator configured to irradiate determination target 102 formed of a resin with light. Near-infrared ray detection device 106 includes a light receiver configured to receive reflected ray 109 from determination target 102 irradiated with light. Conveyor 103 conveys determination target 102. Arithmetic processing device 110 calculates an absorption spectrum of determination target 102 based on reflected ray 109.

In FIG. 6, it can be confirmed that different peaks are provided in a Br-free ABS resin and a Br-containing ABS resin, at a position (1.41 µm) of arrow A and a position (1.43 µm) of arrow B. An absorption spectrum for 1.43 µm is evaluated, and thus it is possible to detect a bromine-based flame retardant and to determine whether or not the bromine-based flame retardant is contained in an ABS resin.

However, in the above determination method, it is not possible to determine whether or not a specific bromine-based flame retardant is contained in a resin.

As a determination method of a specific bromine-based flame retardant for solving the problem, in the related art, a technology of detecting a peak shown in the vicinity of a wave number of 1350 cm⁻¹, by using a Fourier transform infrared spectrophotometry (FT-IR) attenuated total reflection (ATR) method (for example, see Non-Patent Literature 1 ("Analysis of bromine-based flame retardant in plastics" IR/Raman business department of Thermo Fisher Scientific Ltd., Signal-to-News (IR/Raman Customer News Letter), Thermo Fisher Scientific Ltd., 2007, M05011)). FIG. 7 illustrates a graph of an absorption spectrum which is disclosed in Non-Patent Literature 1 and is used for determining whether or not a specific bromine-based flame retardant is contained in polystyrene (PS), by using FT-IR ATR in the related art. The specific bromine-based flame retardant is contained in PS, and thus an unique peak is shown in the vicinity of a wave number of 1350 cm⁻¹. This shown peak is detected, and thus it can be determined whether or not a specific bromine-based flame retardant is contained.

### SUMMARY

According to a specific bromine-based flame retardant determination method of the disclosure, a resin is irradiated with an infrared ray, and a reflected ray from the resin irradiated with the infrared ray is received. In a reflection or absorption spectrum obtained by the reflected ray, a difference of a reflection intensity in a spectrum between a first wave number band of 1340 cm⁻¹ to 1350 cm⁻¹, inclusive, and a second wave number band of 1300 cm⁻¹ to 1340 cm⁻¹, inclusive, is calculated. It is determined whether or not a specific bromine-based flame retardant is contained in the resin, by using the calculated difference of reflection intensity in the spectrum.

According to the disclosure, a specific bromine-based flame retardant determination apparatus includes an irradiator configured to irradiate a resin with an infrared ray, a light receiver configured to receive a reflected ray from the resin irradiated with the infrared ray, and an arithmetic processor configured to calculate a reflection or absorption spectrum of the resin based on the reflected ray, and to calculate a difference of a reflection intensity in a spectrum between a first wave number band of 1340 cm⁻¹ to 1350 cm⁻¹, inclusive, and a second wave number band of 1300 cm⁻¹ to 1340 cm⁻¹,inclusive" so as to determine whether or not a specific bromine-based flame retardant is contained in the resin.

As described above, according to a resin determination method and a determination apparatus according to the aspect of the disclosure, even in a case of a resin such as polypropylene, which has a methyl group, it is possible to evaluate a reflection or absorption spectrum by a specific bromine-based flame retardant, without receiving an influence of absorbing light by the methyl group, and to determine whether or not the specific bromine-based flame retardant is contained, with high accuracy.

### BRIEF DESCRIPTION OF THE DRAWINGS

FIG. 1 is a schematic diagram illustrating a configuration of a specific bromine-based flame retardant determination apparatus according to Exemplary embodiment 1;
FIG. 2 is a graph illustrating a spectrum used for determining whether or not a bromine-based flame retardant is contained in polystyrene, in Exemplary embodiment 1;
FIG. 3 is a graph illustrating a spectrum used for determining whether or not a specific bromine-based flame retardant is contained in polypropylene, in Exemplary embodiment 1;
FIG. 4 is a flowchart illustrating a processing flow of the specific bromine-based flame retardant determination apparatus determining a resin in Exemplary embodiment 1;
FIG. 5 is a schematic diagram illustrating a bromine-based flame retardant determination apparatus in the related art;
FIG. 6 is a graph illustrating an absorption spectrum used for determining whether or not a bromine-based flame retardant is contained in an ABS resin in the related art, which is disclosed in Patent Literature 1; and

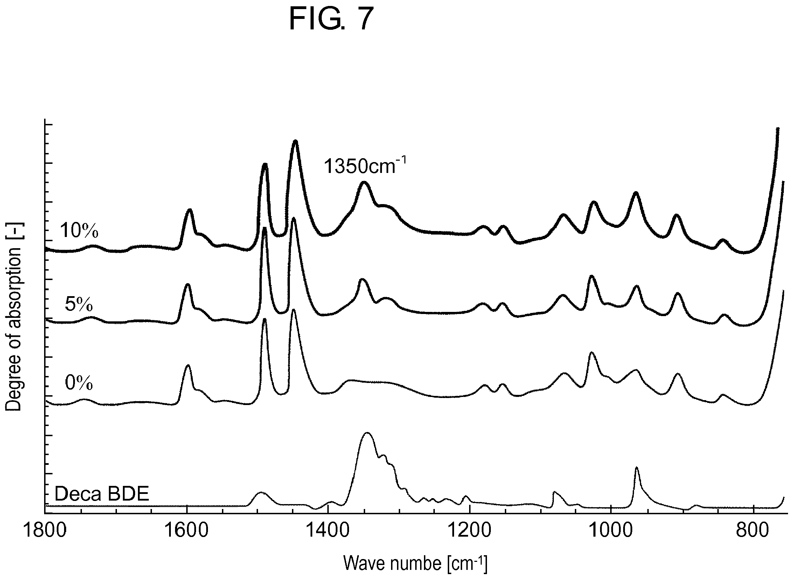

FIG. 7 is a graph illustrating an absorption spectrum used for determining whether or not a specific bromine-based flame retardant is contained in polystyrene by FT-IR ATR in the related art, which is disclosed in Non-Patent Literature 1.

### DETAILED DESCRIPTION

Before an exemplary embodiment is described, a problem in the related art will be simply described.

In a case of using FT-IR, it can be determined whether or not a specific bromine-based flame retardant is contained in ABS or PS. However, in a resin such as polypropylene (PP), which has a methyl group, the methyl group absorbs light in the vicinity of a wave number band in which light is absorbed by a specific bromine-based flame retardant. Thus, a difference of a peak between the specific bromine-based flame retardant and a non-specific bromine-based flame retardant does not occur, and thus it is difficult to determine whether or not the specific bromine-based flame retardant is contained.

To solve the above problem of the related art, an object of the disclosure is to provide a specific bromine-based flame retardant determination method and a determination apparatus that determines whether or not the specific bromine-based flame retardant is contained, with high accuracy even in a case of a resin such as polypropylene, which has a methyl group.

Hereinafter, an exemplary embodiment will be described with reference to the drawings.

### Exemplary embodiment 1

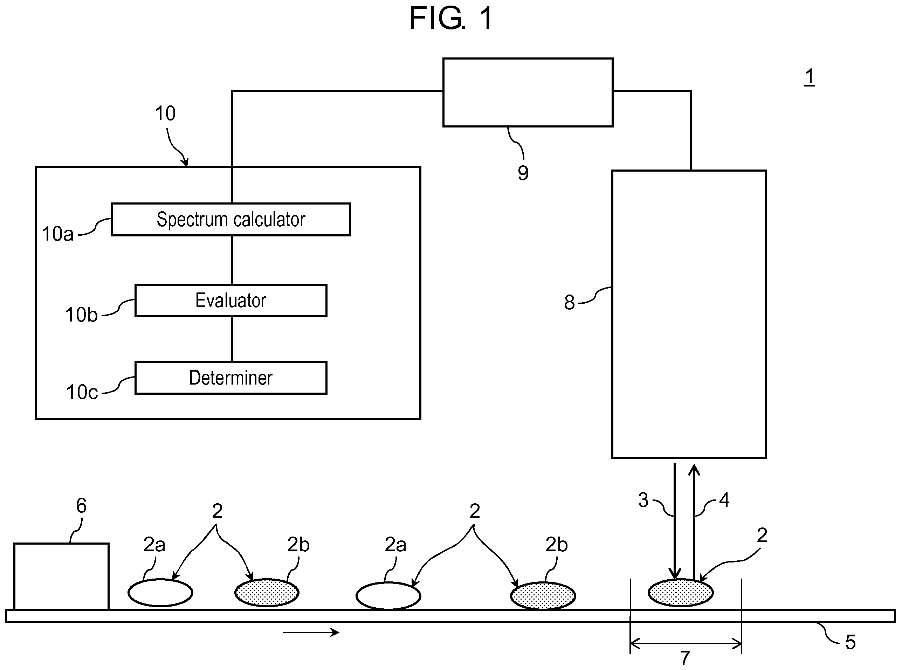

FIG. 1 is a schematic diagram illustrating a configuration of specific bromine-based flame retardant determination apparatus 1 according to Exemplary embodiment 1.

Resin 2 as a determination target substance is a resin such as polypropylene, in which a methyl group is provided and whether or not a specific bromine-based flame retardant is contained is not unclear. A configuration of specific bromine-based flame retardant determination apparatus 1 that detects the specific bromine-based flame retardant from resin 2 will be described with reference to FIG. 1.

Specific bromine-based flame retardant determination apparatus 1 includes at least infrared ray detection unit 8 and arithmetic processing device 10. Specific bromine-based flame retardant determination apparatus 1 may further include digital data conversion device 9.

Infrared ray detection unit 8 includes a function of an irradiator and a light receiver according to the disclosure. Infrared ray detection unit 8 includes a function of irradiating resin 2 with an infrared ray, as the irradiator, and a function of receiving reflected ray 4 from resin 2 of irradiation light 3, and outputting an electrical signal depending on reflected ray 4, as the light receiver.

Digital data conversion device 9 converts the electrical signal output depending on reflected ray 4 by infrared ray detection unit 8, into digital data.

Arithmetic processing device 10 functions as an example of an arithmetic processor according to the disclosure. Arithmetic processing device 10 calculates an absorption spectrum of resin 2, based on the digital data which has been output from digital data conversion device 9.

In FIG. 1, conveyor belt 5 is an example of a conveyor configured to move at a constant speed and convey resin 2 which is a determination target substance. Conveyor belt 5 causes resin 2 to be conveyed from input region 6 to detection region 7 in a longitudinal direction of conveyor belt 5, during a period when resin 2 is put into input region 6 on conveyor belt 5 which moves at a constant speed.

Infrared ray detection unit 8 is disposed over detection region 7 of conveyor belt 5. Thus, infrared ray detection unit 8 can irradiate resin 2 which is a determination target substance reaching detection region 7 of conveyor belt 5, with infrared ray as irradiation light 3, and can receive reflected ray 4 which has been reflected from resin 2 by irradiation of resin 2.

Arithmetic processing device 10 analyzes information output from digital data conversion device 9 and obtains an absorption spectrum of resin 2. Arithmetic processing device 10 evaluates the obtained absorption spectrum so as to detect a specific bromine-based flame retardant. Further, in arithmetic processing device 10, determiner 10c (which will be described later) determines that resin 2 in which a specific bromine-based flame retardant is detected is resin 2a containing the specific bromine-based flame retardant, and determiner 10c determines that resin 2 in which the specific bromine-based flame retardant is not detected is resin 2b which does not contain the specific bromine-based flame retardant.

Here, a method of arithmetic processing device 10 calculating an absorption spectrum from input digital data will be simply described. An electrical signal which is subjected to photoelectric conversion by infrared ray detection unit 8, depending on reflected ray 4, depends on intensity of received light. Thus, in arithmetic processing device 10, information of intensity of reflected ray 4 from resin 2 can be acquired from digital data converted by digital data conversion device 9.

Here, arithmetic processing device 10 includes spectrum calculator 10a, evaluator 10b, and determiner 10c. The absorption spectrum of resin 2 can be acquired from reflection intensity of reflected ray 4, which has been acquired by arithmetic processing device 10, by spectrum calculator 10a. A difference of reflection intensity, for example, a value of a slope (Δreflection intensity /Δwave number) between a plurality of wave number bands is obtained from the absorption spectrum acquired by spectrum calculator 10a. The obtained value of the slope is compared to a preset threshold value range between an upper limit value and a lower limit value. Then, evaluator 10b evaluates whether or not the value of the slope is in the threshold value range, and thus the specific bromine-based flame retardant is detected (evaluated), and determiner 10c determines whether or not the specific bromine-based flame retardant is contained in resin 2, based on the evaluation result of evaluator 10b.

Here, before a method of detecting a specific bromine-based flame retardant, according to Exemplary embodiment 1 is described, absorption of light by a methyl group which is provided in polypropylene and the like, and by the specific bromine-based flame retardant will be described.

In-plane symmetric deformation vibration and out-of-plane antisymmetric deformation vibration of C-H occurs in a methyl group in positive paraffin containing polypropylene. An absorption peak of the in-plane symmetric deformation vibration of C-H is in a wave number of 1379 cm⁻¹, and an absorption peak of the out-of-plane antisymmetric deformation vibration of C-H is in the vicinity of a wave number of 1460 cm⁻¹.

In the specific bromine-based flame retardant, ring stretching vibration causes an absorption peak to be provided by using the vicinity of a wave number of 1350 cm⁻¹ as the center. The ring stretching vibration occurs by a benzene ring which is included in the specific bromine-based flame retardant, and in which H is substituted with Br.

The half-value width of such an absorption peak is more than 10 cm⁻¹ in many cases. Thus, the absorption peak of in-plane symmetric deformation vibration of C-H overlaps the absorption peak by ring stretching vibration of the specific bromine-based flame retardant, and a complex absorption spectrum is shown. Accordingly, it is difficult to determine whether or not the specific bromine-based flame retardant is contained, only by using the absorption peak in the vicinity of the wave number of 1350 cm⁻¹.

The inventors found a method of determining whether or not the specific bromine-based flame retardant is contained even in a case of a resin such as polypropylene, which has a methyl group, that is, a method of detecting a specific bromine-based flame retardant with high accuracy, according to Exemplary embodiment 1.

Next, a result of a test performed based on the principle of the method of detecting a specific bromine-based flame retardant, according to Exemplary embodiment 1 will be described.

A spectrum in a case where a resin containing a specific bromine-based flame retardant was irradiated with an infrared ray having a wave number band of 1300 cm⁻¹ to 1400 cm⁻¹ was obtained.

Firstly, test was performed on a case where resin 2 was polystyrene.

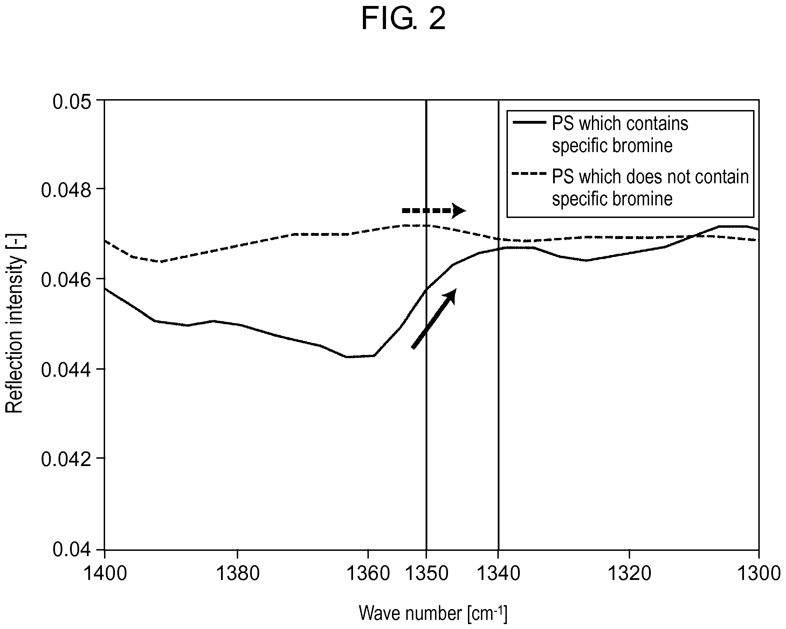

FIG. 2 is a graph illustrating a spectrum in a case where resin 2 is polystyrene. In the graph of FIG 2, a horizontal axis indicates a wave number band and a vertical axis indicates reflection intensity. The followings could be confirmed from the result of FIG. 2. In a case where resin 2 was polystyrene, absorption of light occurs by ring stretching vibration which occurs by a benzene ring in which H is substituted with Br. Thus, attenuation of reflection intensity in polystyrene containing the specific bromine-based flame retardant can be confirmed in the vicinity of a wave number of 1350 cm⁻¹ as the center.

Here, the reason that such attenuation of reflection intensity can be confirmed in the spectrum is because polystyrene does not have a molecular structure indicating significant absorption of light in a wave number band of 1300 cm⁻¹ to 1400 cm⁻¹. It is expected that such a phenomenon similarly occurs in a case where resin 2 is a resin such as ABS resin, which does not have a molecular structure indicating significant absorption of light in a wave number band of 1300 cm⁻¹ to 1400 cm⁻¹, in addition to a case where resin 2 is polystyrene.

Then, regarding a case where resin 2 was polypropylene, a test was performed in order to confirm whether attenuation of reflection intensity occurs in the vicinity of a wave number of 1350 cm⁻¹.

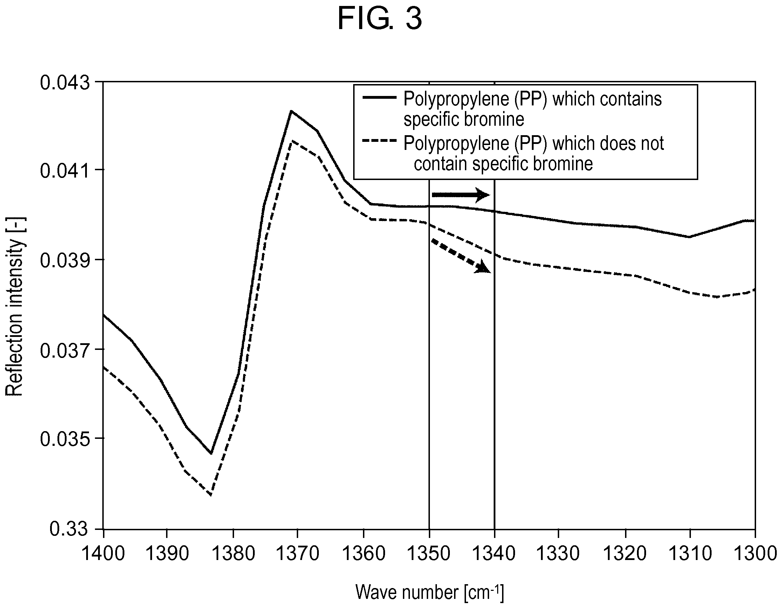

FIG. 3 is a graph illustrating a spectrum in a case where resin 2 is polypropylene. In the graph of FIG 3, a horizontal axis indicates a wave number band and a vertical axis indicates reflection intensity. The followings could be confirmed from the result of FIG. 3. In a case where resin 2 was polypropylene, which was different from a case where resin 2 was polystyrene or an ABS resin, in a wave number of 1350 cm⁻¹ or more, a difference in a spectrum shape was hardly shown between a case of containing specific bromine and a case of not containing specific bromine.

This is because an influence by absorption of light which occurs in a wave number of 1379 cm⁻¹ by in-plane symmetric deformation vibration of C-H of a methyl group provided in polypropylene is large. However, in a wave number band of 1350 cm⁻¹ or less, a difference in a slope of the spectrum can be confirmed. Thus, the inventors found the followings. The spectrum in the first wave number band of 1340 cm⁻¹ to 1350 cm⁻¹, inclusive, and the second wave number band of 1300 cm⁻¹ to 1340 cm⁻¹, inclusive, was evaluated, and thus even though a resin was polypropylene, a spectrum of a resin which contains the specific bromine-based flame retardant is higher than a spectrum of a resin which does not contain the specific bromine-based flame retardant. Thus, it could be determined whether or not the specific bromine-based flame retardant was contained.

In a case where the type of a resin is known, the spectrum in the first wave number band of 1340 cm⁻¹ to 1350 cm⁻¹,inclusive, is evaluated, and thus a spectrum of a resin containing the specific bromine-based flame retardant is lower than a spectrum of a resin which does not contain the specific bromine-based flame retardant, even in a case of polystyrene or an ABS resin in addition to only a resin such as polypropylene, which includes a methyl group. Accordingly, it is possible to determine whether or not the specific bromine-based flame retardant is contained.

Next, the method of detecting a specific bromine-based flame retardant will be described by using specific bromine-based flame retardant determination apparatus 1.

It is assumed that two light sources, that is a first light source which can irradiate resin 2 with light of a wave number of 1340 cm⁻¹ to 1350 cm⁻¹,inclusive, and a second light source which can irradiate resin 2 with light of a wave number of 1300 cm⁻¹ to 1340 cm⁻¹, inclusive, are provided as an example of the irradiator in infrared ray detection unit 8. It is assumed that a first light receiving element and a second light receiving element that respectively receive reflected ray 4 from the two light sources of the first light source and the second light source are provided as a light receiving element of the light receiver in infrared ray detection unit 8. In arithmetic processing device 10, spectrum calculator 10a calculates a first reflection intensity corresponding to a first wave number and a second reflection intensity corresponding to a second wave number in the two wave numbers of the first wave number and the second wave number of resin 2, that is, calculates a first spectrum and a second spectrum, based on an output from the two elements of the first light receiving element and the second light receiving element. Evaluator 10b performs evaluation, and thus determiner 10c determines whether or not the specific bromine-based flame retardant is contained. Specifically, a slope (Δreflection intensity /Δwave number) is calculated from two wave numbers of the two light sources of the first light source and the second light source, and first reflection intensity and second reflection intensity thereof. Then, a determination algorithm in which determiner 10c compares a numerical value of a result obtained by calculation to a preset threshold value range between an upper limit value and a lower limit value is performed, and thus determiner 10c determines whether or not the specific bromine-based flame retardant is contained. For example, if the numerical value of the result obtained by calculation is in the threshold value range, determiner 10c determines that the specific bromine-based flame retardant is contained. If the numerical value of the result obtained by calculation is not in the threshold value range, determiner 10c determines that the specific bromine-based flame retardant is not contained.

Here, the method using a slope (Δreflection intensity /Δwave number) is described as the determination algorithm. However, for example, a method of obtaining a ratio of reflection intensity (reflection intensity in one light source/reflection intensity in another light source) or obtaining a difference of reflection intensity (Δreflection intensity) is appropriately selected.

The method using the two light receiving elements which respectively correspond to the two light sources, as a light source is described. However, three or more light sources and light receiving elements which respectively correspond to the light sources can be used. For example, a method using regression analysis is appropriately selected as a determination algorithm in this case.

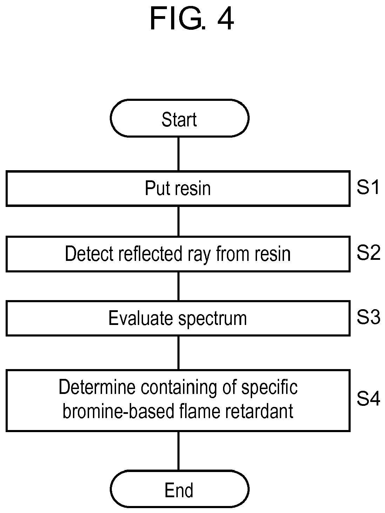

Next, an operation of specific bromine-based flame retardant determination apparatus 1 in FIG. 1 will be described with reference to the flowchart in FIG. 4.

Here, focusing on one resin 2 among a plurality of provided resins, a flow until it is determined whether or not the specific bromine-based flame retardant is contained will be described. It is assumed that resin 2 as a determination target substance is polypropylene.

Firstly, in Step S1, resin 2 is put into input region 6 on conveyor belt 5 which moves a constant speed.

Next, in Step S2, infrared ray detection unit 8 irradiates resin 2 reaching detection region 7 in conveyor belt 5 with an infrared ray, detects reflected ray 4 from resin 2, and outputs an electrical signal depending on reflected ray 4.

Then, in Step S3, information of reflected ray 4, which has been detected by infrared ray detection unit 8 is output to arithmetic processing device 10 from infrared ray detection unit 8 through digital data conversion device 9. In arithmetic processing device 10, spectrum calculator 10a calculates a spectrum of resin 2 based on the input information of reflected ray 4.

A value of a slope (Δreflection intensity /Δwave number) between the first wave number band and the second wave number band which are described above is obtained from the spectrum calculated by spectrum calculator 10a. For example, a first wave number and first reflection intensity in the first wave number band of 1340 cm⁻¹ to 1350 cm⁻¹, inclusive, are obtained, and a second wave number and second reflection intensity in the second wave number band of 1300 cm⁻¹ to 1340 cm⁻¹, inclusive, are obtained. (Δreflection intensity /Δwave number)={(the first reflection intensity - the second reflection intensity)/(the first wave number - the second wave number)} is obtained as the value of the slope between the first wave number band and the second wave number band. Then, the obtained value is compared to the preset threshold value range between an upper limit value and a lower limit value, and evaluator 10b evaluates whether or not the value of the slope is in the threshold value range.

Then, in Step S4, determiner 10c determines whether or not the specific bromine-based flame retardant is contained in resin 2, based on evaluation of the value of the slope and the threshold value range in evaluator 10b of arithmetic processing device 10. Specifically, evaluator 10b compares the value of the slope obtained by spectrum calculator 10a, to the preset threshold value range between an upper limit value and a lower limit value. When evaluator 10b evaluates that the value of the slope is in the threshold value range, determiner 10c determines that resin 2 of the determination target substance is a resin which contains the specific bromine-based flame retardant. When evaluator 10b evaluates that the value of the slope is not in the threshold value range, determiner 10c determines that resin 2 of the determination target substance is a resin which does not contain the specific bromine-based flame retardant.

As described above, according to the specific bromine-based flame retardant determination method and the determination apparatus according to Exemplary embodiment 1, it is possible to determine whether or not the specific bromine-based flame retardant is contained in resin 2, based on a difference of reflection intensity in a spectrum between a first wave number band of 1340 cm⁻¹ to 1350 cm⁻¹,inclusive, and a second wave number band of 1300 cm⁻¹ to 1340 cm⁻¹,inclusive, in a reflection or absorption spectrum obtained by reflected ray 4 from resin 2. Thus, even though the resin is a resin such as polypropylene, which has a methyl group, it is possible to evaluate a reflection or absorption spectrum by a specific bromine-based flame retardant, without receiving an influence of absorbing light by the methyl group, and to determine whether or not the specific bromine-based flame retardant is contained, with high accuracy.

A certain exemplary embodiment or modification example among various exemplary embodiments or modification examples are appropriately combined, and thus effects provided in the exemplary embodiment and modification example can be shown. A combination of exemplary embodiments, a combination of examples, or a combination of an exemplary embodiment and an example may be made, and a combination of features in different exemplary embodiments or examples may be made.

The specific bromine-based flame retardant determination method and determination apparatus according to the exemplary embodiment of the disclosure can rapidly determine whether or not a specific bromine-based flame retardant is contained in a mixture of plural types of resins even in a case of a resin such as polypropylene, which has a methyl group. Thus, the specific bromine-based flame retardant determination method and determination apparatus can be used in a recycling process and the like of rapidly determining a plurality of determination target substances. (arithmetic processor).

## Claims

1. A specific bromine-based flame retardant determination method comprising:
irradiating a resin with an infrared ray;
receiving a reflected ray from the resin irradiated with the infrared ray;
calculating a difference of a reflection intensity in a spectrum between a first wave number band of 1340 cm⁻¹ to 1350 cm⁻¹,inclusive, and a second wave number band of 1300 cm⁻¹ to 1340 cm⁻¹,inclusive, in a reflection or absorption spectrum obtained by the reflected ray; and
determining whether or not a specific bromine-based flame retardant is contained in the resin by using the difference of the reflection intensity in the spectrum.

2. The specific bromine-based flame retardant determination method of Claim 1,
wherein the resin is a resin including a methyl group,
in the calculating, a difference of the reflection intensity of the spectrum between the first wave number band and the second wave number band is calculated by (i) obtaining a first wave number in the first wave number band of 1340 cm⁻¹ to 1350 cm⁻¹,inclusive, and a first reflection intensity corresponding to the first wave number, (ii) obtaining a second wave number in the second wave number band of 1300 cm⁻¹ to 1340 cm⁻¹,inclusive, and a second reflection intensity corresponding to the second wave number, and (iii) obtaining (Δ reflection intensity /Δ wave number)={(the first reflection intensity - the second reflection intensity)/(the first wave number - the second wave number)} as a value of a slope between the first wave number band and the second wave number band, and
the determining of whether or not the specific bromine-based flame retardant is contained in the resin includes:
comparing the obtained value of the slope to a threshold value range which is preset; and
determining that the resin contains the specific bromine-based flame retardant when the value of the slope falls within the threshold value range, and that the resin does not contain the specific bromine-based flame retardant when the value of the slope falls outside the threshold value range.

3. A specific bromine-based flame retardant determination apparatus comprising:
an irradiator configured to irradiate a resin with an infrared ray;
a light receiver configured to receive a reflected ray from the resin irradiated with the infrared ray; and
an arithmetic processor configured to calculate a reflection or absorption spectrum of the resin based on the reflected ray, to calculate a difference of a reflection intensity in a spectrum between a first wave number band of 1340 cm⁻¹ to 1350 cm⁻¹, inclusive, and a second wave number band of 1300 cm⁻¹ to 1340 cm⁻¹, inclusive, in the spectrum, so as to determine whether or not a specific bromine-based flame retardant is contained in the resin.

4. The specific bromine-based flame retardant determination apparatus of Claim 3,
wherein the resin is a resin including a methyl group, and
the arithmetic processor includes
a spectrum calculator configured to obtain a first wave number in the first wave number band of 1340 cm⁻¹ to 1350 cm⁻¹,inclusive, and a first reflection intensity corresponding to the first wave number, and to obtain a second wave number in the second wave number band of 1300 cm⁻¹ to 1340 cm⁻¹,inclusive, and a second reflection intensity corresponding to the second wave number,
an evaluator configured to obtain (Δ reflection intensity /Δ wave number)={(the first reflection intensity - the second reflection intensity)/(the first wave number - the second wave number)} as a value of a slope between the first wave number band and the second wave number band, so as to calculate a difference of the reflection intensity of the spectrum between the first wave number band and the second wave number band, and to compare the obtained value of the slope to a threshold value range which is preset, and
a determiner configured to determine that the resin is contains the specific bromine-based flame retardant when the value of the slope falls within the threshold value range, and that the resin does not contain the specific bromine-based flame retardant when the value of the slope falls outside the threshold value range.
